# EUROPÄISCHE PATENTSCHRIFT

(11) **EP 1 023 052 B1**
(45) Veröffentlichungstag und Bekanntmachung des Hinweises auf die Patenterteilung: **10.09.2003**
(21) Anmeldenummer: 98955425.8
(22) Anmeldetag: 13.10.1998
(51) Int. Cl.: A61K 9/51

(54) **ARZNEISTOFFTRÄGERPARTIKEL FÜR DIE GEWEBESPEZIFISCHE ARZNEISTOFFAPPLIKATION**
MEDICAMENT EXCIPIENT PARTICLES FOR TISSUE-SPECIFIC APPLICATION OF A MEDICAMENT
PARTICULES D'EXCIPIENT DE MEDICAMENT CONVENANT A L'APPLICATION D'UN MEDICAMENT PROPRE AUX TISSUS

(30) Priorität: 17.10.1997 DE 19745950
(43) Veröffentlichungstag der Anmeldung: 02.08.2000
(60) Teilanmeldung: 03000323.0
(73) Patentinhaber: DDS DRUG DELIVERY SERVICE GESELLSCHAFT ZUR FÖRDERUNG DER FORSCHUNG IN PHARMAZEUTISCHER TECHNOLOGIE UND BIOPHARMAZIE MBH, 24119 Kronshagen (DE)
(72) Erfinder: MÜLLER, Rainer, H., D-12161 Berlin (DE); LÜCK, Martin, D-10965 Berlin (DE); KREUTER, Jörg, D-61350 Bad Homburg (DE)
(74) Vertreter: UEXKÜLL & STOLBERG
(86) Internationale Anmeldenummer: EP9806429
(87) Internationale Veröffentlichungsnummer: WO99020256

(56) Entgegenhaltungen:
- EP-A- 0 671 169
- EP-B- 0 406 162
- EP-B- 0 547 191
- WO-A-87/02061
- WO-A-91/04753
- WO-A-93/00076
- WO-A-95/00126
- WO-A-95/26376
- WO-A-98/16202
- WO-A-99/08701
- WO-A-99/16460
- DE-A- 4 018 325
- DE-A- 4 310 935
- DE-A- 19 745 950
- BLUNK T ET AL.: "Kinetics of plasma protein adsorption on model particles for controlled drug delivery and drug targeting" EUROPEAN JOURNAL OF PHARMACEUTICS AND BIOPHARMACEUTICS, Bd. 42, Nr. 4, 1996, Seiten 262-268, XP002102920
- MÜLLER R H ET AL.: "Intravenously injected particles. Surface properties and interaction with blood proteins - the key determining the organ distribution. In: Scientific and Clinical Applications of Magnetic Carriers" 1997 , PLENUM PRESS , NEW YORK XP002102925 siehe das ganze Dokument
- ALYAUTDIN R N ET AL.: "Delivery of loperamide across the blood-brain barrier with polysorbate 80-coated polybutylcyanoacrylate nanoparticles " PHARMACEUTICAL RESEARCH, Bd. 14, Nr. 3, 1997, Seiten 325-328, XP002102921 in der Anmeldung erwähnt
- TRÖSTER S D ET AL.: "Modification of the body distribution of poly(methyl methacrylate) nanoparticles in rats by coating with surfactants" INTERNATIONAL JOURNAL OF PHARMACEUTICS, Bd. 61, 1990, Seiten 85-100, XP002102922 in der Anmeldung erwähnt
- ALYAUTDIN R ET AL: "ANALGESIC ACTIVITY OF THE HEXAPEPTIDE DALARGIN ADSORBED ON THE SURFACE OF POLYSORBATE 80-COATED POLY(BUTYL CYANOACRYLATE) NANOPARTICLES" EUROPEAN JOURNAL OF PHARMACEUTICS AND BIOPHARMACEUTICS, Bd. 41, Nr. 1, 1. Januar 1995, Seiten 44-48, XP000482891
- MÜLLER R H ET AL.: "Solid lipid nanoparticles (SLN) as potential carrier for human use: interaction with human granulocytes" JOURNAL OF CONTROLLED RELEASE, Bd. 47, 1997, Seiten 261-269, XP002102923
- KREUTER J: "NANOPARTICLE-BASED DRUG DELIVERY SYSTEMS" JOURNAL OF CONTROLLED RELEASE, Bd. 16, Nr. 1 / 02, 1. Juni 1991, Seiten 169-176, XP000219660
- LÜCK M ET AL.: "Plasma protein adsorption on biodegradable microspheres consisting of poly(D,L-lactide-co-glycolide), poly(L-lactide) or ABA triblock copolymers containing poly(oxyethylene). Influence of production method and polymer composition" JOURNAL OF CONTROLLED RELEASE, Bd. 55, 1998, Seiten 107-120, XP002102924
- KREUTER J ET AL: "INFLUENCE OF THE TYPE OF SURFACTANT ON THE ANALGESIC EFFECTS INDUCED BY THE PEPTIDE DALARGIN AFTER ITS DELIVERY ACROSS THE BLOOD-BRAIN BARRIER USING SURFACTANT-COATED NANOPARTICLES" JOURNAL OF CONTROLLED RELEASE, Bd. 49, Nr. 1, 10. November 1997, Seiten 81-87, XP000667154
- C.L. BISGAIER: "Effects of Apolipoproteins A-IV and A-I on the uptake of phospholipid liposomes by hepatocytes", J. BIOL. CHEM., 1989, Band 264, Nr. 2, Seiten 862 - 866
- B. LUNDBERG ET AL.: "Conjugation of Apolipoprotein B with liposomes and targeting to cells in culture", BIOCHEM. BIOPHYS. ACTA, 1993, Band 1149, Nr. 2, Seiten 305 - 312
- P. C. N. RENSEN: "Human recombinant Apolipoprotein E-enriched Liposomes can mimic LDL as carriers for the site-specific delivery of antitumor agents", MOLECULAR PHARMACOLOGY, 1997, Band 53, Nr. 3, Seiten 445 - 455
- M. MASQUELIER, S. VITOLS, C. PETERSON: "Low-density Lipoprotein as a carrier of antitumoral drugs", CANCER RESEARCH, 1986, Band 46, Seiten 3842 - 3847

## Beschreibung

Die Erfindung betrifft Arzneistoffträgerpartikel, die für die Gewebespezifische Arzneistoffappplikation, speziell zum zentralen Nervensystem (ZNS), geeignet sind.

Die Therapie von Erkrankungen des ZNS wird durch die Blut-Hirn-Schranke, einer der wichtigsten und undurchlässigsten physiologischen Barrieren im Organismus, erschwert. Als morphologisches Substrat der Blut-Hirn-Schranke wird in erster Linie das Gefäßendothel der Hirnkapillaren angesehen, da die Interzellulärspalten zwischen den Endothelzellen durch dichte Zell-Zell-Verbindungen ("tight junctions") überbrückt sind. Die Endothelzellen sind darüber hinaus von einer lückenlosen Basalmembran umschlossen. Typisch für das Gewebe sind die fehlende Fenestrierung, die Abwesenheit von Poren und eine geringe pinozytotische Aktivität. Hinzu kommt, daß die Blutgefäße im Bereich des ZNS von einer dicht anliegenden Schicht von Gliazellen eingehüllt sind (Thews, G., Mutschler, E., Vaupel, P., *Anatomie, Physiologie und Pathophysiologie des Menschen,* 3. Aufl., Wissenschaftliche Verlagsgesellschaft, Stuttgart, 1989; Borchard, G., in: Müller, R. H., Hildebrand, G. (Hrsg.), *Pharmazeutische Technologie: Moderne Arzneiformen*. Wissenschaftliche Verlagsgesellschaft, Stuttgart, 1997, 291-296). Das Gehirn kann daher vom Blut aus in der Regel nur von lipophilen Pharmaka mit einem geringen Molekulargewicht (MG < 500) erreicht werden (Pardridge, W. M., *J. Control. Rel*., 39, 281-286, 1996).

Für sehr viele Wirkstoffe wie z.B. Peptide, Proteine und Oligonukleotide als mögliche Therapeutika für Krankheiten des ZNS ist die Blut-Hirn-Schranke normalerweise nicht permeabel.

Nach Pardridge (*J. Control. Rel*., 39, 281-286, 1996) können die Strategien für einen Arzneistofftransport ins Gehirn eingeteilt werden in
a) invasive,
b) pharmakologische und
c) physiologische Verfahren.

Mit invasiven Techniken kann die Blut-Hirn-Schranke physikalisch umgangen werden, indem z.B. ein Arzneistoffträgersystem ins Gehirn implantiert wird (Domb, A. J., Ringel, I., in: Flanagan, T. R., Emerich, D. F., Winn, S. R. (Hrsg.), *Providing Pharmacological Access to the Brain.* Academic Press, Inc., New York, 1994, 169-187; Friden, P. M., *J. Control. Rel*., 46, 117-128, 1996). Nachteilig bei diesen Techniken ist, daß sie mit einem chirurgischen Eingriff verbunden sind und sich deshalb nicht als gängige Behandlungsmethode etabliert haben.

Die pharmakologischen Strategien für einen Arzneistofftransport durch die Blut-Hirn-Schranke umfassen Maßnahmen zur Erhöhung der Lipophilie von Arzneistoffen (Chekhonin, V. P., Kabanov, A. V., Zhirkov, Y. A., Morozov, G. V., *FEBS Lett.,* 287, 149-152, 1991). Nachteile dieser Verfahren sind, daß "new drug entities" entstehen, für die umfangreiche kostenintensive toxikologische Untersuchungen gemacht werden müssen, daß diese Verfahren nur für relativ kleine Moleküle praktikabel sind und daß sie eine geringe Effizienz besitzt (Friden, P. M., *J. Control. Rel*., 46, 117-128, 1996; Pardridge, W. M., *J. Control. Rel*., 39, 281-286, 1996).

Physiologische Strategien für einen Arzneistofftransport ins Gehirn basieren auf der Kenntnis spezieller aktiver,spezifischer Transportmechanismen an der Blut-Hirn-Schranke z.B. für Nährstoffe (u.a. Glucose und Aminosäuren), Peptide oder Proteine (Pardridge, W. M., *Peptide Drug Delivery to the Brain,* Raven Press, New York, 1991; Pardridge, W. M., *J. Control. Rel*., 39, 281-286, 1996; Friden, P. M., *J. Control. Rel*., 46, 117-128, 1996). Ein Beispiel ist L-Dopa als Vorstufe des Neurotransmitters Dopamin, welcher die Blut-Hirn-Schranke nicht zu überwinden vermag. L-Dopa hingegen wird durch einen aktiven Transportweg für neutrale Aminosäuren ("Neutrale Aminosäuren-Carrier") durch die Blut-Hirn-Schranke in die Zellen des Gehirns transportiert, wo die eigentliche Wirkform Dopamin gebildet wird (Mutschler, E., *Arzneimittelwirkungen, Lehrbuch der Pharmakologie und Toxikologie*, 7. Aufl., Wissenschaftliche Verlagsgesellschaft, Stuttgart, 1996; Borchard, G., in: Müller, R. H., Hildebrand, G. (Hrsg.), *Pharmazeutische Technologie: Moderne Arzneiformen*. Wissenschaftliche Verlagsgesellschaft, Stuttgart, 1997, 291-296). Aber auch dieser Ansatz hat sich aufgrund folgender Nachteile nicht auf breiter Ebene durchgesetzt:
1. die aktiven Transportmechanismen sind sehr Substrat-spezifisch, d.h., nur wenige Arzneistoffe mit großer Ähnlichkeit zum Substrat werden transportiert, was die Einsatzfähigkeit dieser Strategie sehr limitiert.
2. Konjugate aus natürlichem Substrat und Arzneistoff werden wegen der großen Spezifität des Transporters (chemische Struktur und dreidimensionale Struktur und Größe des zu transportierenden Substrats) nicht oder wenig effizient transportiert.

Ein weiterer Ansatz für die gewebespezifische Arzneistoffapplikation, z.B. ins ZNS, ist die Einarbeitung von Arzneistoffen in partikuläre Arzneistoffträger wie Nanopartikel, Mikropartikel, Emulsionen und Liposomen sowie Verarbeitung zu partikulären Arzneiformen wie Hydrosole, Nanokristalle und Nanosuspensionen. Generell ist für intravenös injizierte Partikel die Überwindung von Endothelien aufgrund ihrer Größe (in der Regel >> 30 nm) noch schwieriger als für Arzneistoffmoleküle (Größe im Ångström-Bereich). So wird beispielsweise für Liposomen generell eine sehr begrenzte Fähigkeit zur Penetration durch die Blut-Hirn-Schranke beschrieben (Gennuso, R., Spigelman, M. K., Chinol, M., Zappulla, R. A., Nieves, J., Vallabhajosula, S., Paciucci, P. A., Goldsmith, S. J., Holland, J. F., *Cancer Invest*., 11, 118-128, 1993; Boado, R. J., *Adv. Drug Deliv. Rev.,* 15, 73-107, 1995; Boado, R. J., *Proceed. Intern. Symp. Control. Rel. Bioact. Mater.,* 24, 223-224, 1997; Pardridge, W. M., *J. Control. Rel*., 39, 281-286, 1996).

Einen ersten Erfolg hinsichtlich der Applikation eines Arzneistoffs zum ZNS mit partikulären Trägern publizierten Alyautdin et al. (Alyautdin, R. N., Gothier, D., Petrov, V. E., Kharkevich, D. A., Kreuter, J., *Eur. J. Pharm. Biopharm*., 41, 44-48, 1995). Sie demonstrierten für i.v. applizierte Polybutylcyanoacrylat(PBCA)-Nanopartikel, auf deren Oberfläche die analgetisch wirksame Substanz Dalargin durch Adsorption gebunden wurde, einen dosisabhängigen analgetischen Effekt im "tail-flick-Test" an Mäusen. Das Hexapeptid Dalargin (Tyr-D-Ala-Gly-Phe-Leu-Arg) ist ein Leu-Enkephalin-Analogon und besitzt als Opioid-Rezeptor-Agonist eine zentral analgetische Wirkung. Dalargin kann normalerweise die Blut-Hirn-Schranke nicht überwinden.

Eine i.v. Applikation von Dalargin führt trotz der Stabilität im Blut auch in hoher Dosierung (20 mg/kg) zu keinem analgetischen Effekt (Kalenikova, E. I., Dmitrieva, O. F., Korobov, N. N., Zhukova, S. V., Tischenko, V. A., *Vopr. Med. Khim*., 34, 75-83, 1988).

In einer anderen Studie konnte im Rattenmodell nach intravenöser Injektion von oberflächenmodifizierten Polymethylmethacrylat-(PMMA)-Nanopartikeln eine Anreicherung der Partikel im Gehirnbereich detektiert werden (Tröster, S. D., Müller, U., Kreuter, J., *Int. J. Pharm*., 61, 85-100, 1990). Die Autoren hielten es aber für ausgeschlossen, daß die Partikel in Gehirnzellen aufgenommen werden, was eine Applikation von Arzneistoff ins Gehirn ausschließt.

Nachteilig ist, daß das von Alyautdin et al. *(Eur. J. Pharm. Biopharm*., 41, 44-48, 1995) und von Schröder und Sabel (*Brain Res.,* 710, 121-124, 1996) berichtete Phänomen nicht für eine gezielte und kontrollierte Arzneistoffapplikation benutzt werden kann. Der Mechanismus ist nicht bekannt. Es bleibt nur die "Trial-und-Error-Vorgehensweise", um zu detektieren, ob eine Zumischung eines Tensids zu einem partikulären Träger vielleicht zufällig eine Anreicherung im Gehirn erzeugt. Die Wahrscheinlichkeit daß es dazu kommt ist gering, da Tenside oft in Partikelpräparationen eingesetzt wurden (Couvreur, P., Dubernet, C., Puisieux, F., *Eur. J. Pharm. Biopharm*., 41, 2-13, 1995) und bisher die obigen Berichte die ersten Daten über eine Aufnahme eines Arzneistoffes in das Gehirn sind.

### Stand der Tecknik

Die WO 99/16460 offenbart Komplexe von Apolipoprotein E und Nervenwachstumsfaktor, Neurotrophin 4 oder γ-Interferen wobei die Bindung Kovalent ist. (Stand der Technik nach Art 54(3) EPÜ).

Die WO 99/08 701 offenbart Komplexe von Apolipoprotein E und ciliarem neurotrophen Faktor, wobei diese Bindung ebenfalls kovalent ist (Stand der Technik nach Art. 54(3) EPÜ).

Die WO 87/02061 offenbart eine Zusammen setzung zur Abgabe eines Wirkstoffes, die ein gewebsspezifisches Peptid umfaßt, das eine LDL-Rezeptor bindende Region aufweist, die Kovalent an eine Substanz gebunden ist, die ein Werkstoff sein kann, wobei die LDL - Rezeptor-bindende Region sich bei Spielsweise von Apo E ableitet.

Die DE 40 18 325 A1 offenbart biotinylierte Apoprotein, u. a. Apo E, die in liposamen integriert sein können.

Für einen Arzneistofftransport spezifisch in das gewünschte Zielgewebe, insbesondere auch ins Gehirn, wäre eine Arzneiform optimal, die
1. die Spezifität eines Transportweges beispielsweise über Rezeptor-vermittelte Transzytose (physiologische Strategie) mit der hohen Transportkapazität partikulärer Arzneistoffträger, z.B. Nanopartikel, verbindet,
2. die Aufnahme des Arzneistoffes in das Gewebe - z.B. das Gehirn - über ein generell einsetzbares Erkennungsmölekül ermöglicht und
3. eine kontrollierte Befestigung des Erkennungsmoleküls an die Oberfläche von Partikeln erlaubt.

Der Vorteil von partikulären Arzneistoffträgern - im Gegensatz zu z.B. Molekülkonjugaten - liegt neben der hohen Transportkapazität in der Möglichkeit, über die Wahl der Trägermatrix (z.B. Polymer, Lipid) und der Herstellungsbedingungen der Partikel viele in ihren physikochemischen Eigenschaften und ihrem Molekulargewicht unterschiedliche Arzneistoffe transportieren zu können (Borchard, G., in: Müller, R. H., Hildebrand, G. (Hrsg.), *Pharmazeutische Technologie: Moderne Arzneiformen.* Wissenschaftliche Verlagsgesellschaft, Stuttgart, 1997, 291-296).

Der Erfindung liegt daher die Aufgabe zugrunde, Arzneistoffträgerpartikel zu schaffen, die in der Lage sind, die Blut-Hirn-Schranke zu überwinden und gewünschte Arzneiwirkstoffe in das ZNS einzuführen.

Diese Aufgabe wird gemäß dem Ansprüchen 1 und 15 gelöst.

Bevorzugte Ausführungsformen sind Gegenstand der Unteransprüche.

Als Partikelmaterial sind PBCA- Polymer, der Arzneistoff selbst in Form von Nanosuspensionen bz.w. Hydrosolen oder feste Lipide geeignet.

Arzneistoffträgerpartikel, die durch Bindung des (auch natürlich im Blut vorkommenden) Erkennungsproteins Apolipoprotein E, an die Partikeloberfläche, ggf. im Kombination mit eimen oder mehreren Erkennungsproteinen, modifiziert sind, können Arzneistoffe spezifisch zum Zielgewebe transportieren, insbesondere zum Zentralen Nervensystem (ZNS). Die Erkennungsproteine werden an die Partikeloberfläche adsorbiert, kovalent daran gebunden oder durch gezielte Modifikation der Partikeloberfläche (chemisch, physikalisch, Adsorption von die Adsorption des Erkennungsproteins vermittelnden Molekülen) präferentiell adsorbiert.

Die Adsorption der Erkennungsproteine kann vor Applikation der Arzneistoffträger erfolgen oder - nach entsprechender Modifikation der Oberfläche der Arzneistoffträger - auch in vivo im Körper. Im Körper vorhandene, physiologische Erkennungsproteine, insbesondere Apolipoprotein E, adsorbieren präferentiell auf applizierten Arzneistoffträgern, insbesondere auf mit speziellen Tensiden oder Polymeren modifizierten Trägern, die beispielsweise durch intravenöse Applikation in den Körper eingebracht worden sein können.

Das Erkennungsprotein Apo-E kann durch unspezifische oder spezifische Adsorption an die Oberfläche der Partikel gebunden sein.

Ferner kann das Erkennungsprotein kovalent an die Oberfläche der Partikel gebunden sein. Dabei wird worzugsweise eine Bindung an Partikel mit reaktiven Oberflächengruppen, insbesondere Epoxyoder Aldehydgruppen, oder nach Aktivierung der Partikeloberfläche mit Aktivatoren, insbesondere Carbodiimid, N-Ethoxycarbonyl-2-ethoxy-1,2-dihydrochinolin, Glutardialdehyd, Bromzyan, meta-Perjodat (Na-Salz oder K-Salz), Tosylchlorid und Chlorameisensäureester bewirkt. Dabei kann die Bindung der Erkennungsproteine über ihre Aminogruppen bewirkt werden.

Außerdem kann das Erkennungsprotein durch präferentielle Adsorption an die Oberfläche der Partikel gebunden sein. Dabei kann die präferentielle Adsorption aus Proteinlösungen oder durch Kontakt der Partikel mit Plasma, Serum oder Blut erfolgen, wobei letzteres auch ex vivo oder in vivo erfolgen kann.

Die Oberfläche der Partikel kann vorzugsweise vor der präferentiellen Adsorption durch Einführung funktioneller Gruppen, insbesondere Hydroxyl-, Carboxyl-, Amino-, Hydroxyethyl, Epoxy oder Aldehydgruppen und deren Derivate chemisch modifiziert werden oder durch physikalische Behandlung mit Plasma, insbesondere plasma etching, zur Einführung von Hydroxylgruppen in den Eigenschaften verändert werden.

Ferner kann die Oberfläche durch Adsorption von Substanzen modifiziert werden, die zu einer präferentiellen Adsorption des Erkennungsproteins führen, wobei die modifizierende Substanz im Verhältnis zum Arzneistoffpartikel in einer gewichtsbezogenen Menge von 0.01 Teile bis 10 modifizierende Substanz pro 1 Teil Partikel, vorzugsweise 0,1 bis 10 Teile modifizierende Substanz pro 1 Teil Partikel, und insbesondere 1 Teil modifizierende Substanz pro 1 Teil Partikel eingesetzt wird.

Geeignete Substanzen umfassen insbesondere Tenside, speziell ethoxylierten Tenside, vorzugsweise Polyethylenglykol-Fettsäureester und Polyethylenglykol-Fettalkoholether, bevorzugt Polyetylenglykol-Sorbitanfettsäureester und Polyethylenglykol-Fettsäureglyceride, bevorzugter Tween® 20, 40, 60 und 80 oder Cremophor EL® und RH40.

Geeignet sind beispielsweise auch Polymere, insbesondere Polymere aus den Poloxameren und Poloxaminen, Cellulosen und ihren Derivaten, vorzugsweise Methylcellulose, Hydroxyethylcellulose, Hydroxypropyl-Methylcellulose, Hydroxypropylcellulose, Carboxymethylcellulose-Natrium sowie Xanthan, Polyvinylalkohol, Polyvinylpyrrolidon, Polyacrylsäuren, Polyethylenglykolen, Polyethylenglykol-enthaltenden Block-Copolymeren, Stärke und -derivaten, Dextran und -derivaten, Polyethylenimin und Gelatine.

Das Erkennungsprotein ist Apolipoprotein E, das in Kombination mit einem oder mehreren anderen Erkennungsproteinen vorliegen kann, insbesondere mit Apolipoprotein A-I, A-II, A-IV, B, C-II, C-III, D, H und/oder J, und/oder mit Albumin.

Apo C-II, Apo C-III, Apo A-IV, Apo-E werden vorzugsweise einzeln oder in Kombinationen von 2 oder 3 oder 4 Apolipoproteinen eingesetzt.

Das Erkennungsprotein wird, bezogen auf den Arzneistoffträger, im allgemeinen in einer Menge von 0,001 bis 40 Gew.-%, insbesondere von 0,01 bis 30 Gew.-% und bevorzugt von 0,1 bis 15 Gew.-% eingesetzt.

Für Apolipoprotein (Apo) E existieren verschiedene Rezeptoren, die zu der Familie der LDL-Rezeptoren gehören (Schneider, W. J., Nimpf, J., *Curr. Opin. Lipid*., 4, 205-209, 1993).

ApoE-Rezeptoren finden sich ebenfalls an der Blut-Hirn-Schranke. Im Liquor cerebrospinalis wird die Aufnahme von Lipoproteinen über zelluläre LDL-Rezeptoren nach bisherigen Erkenntnissen ausschließlich von ApoE vermittelt. ApoE wird vorwiegend in den Astrozyten synthetisiert und abgesondert. Diese Zellen exprimieren auch LDL-Rezeptoren (Boyles, J. K., Pitas, R. E., Wilson, E., Mahley, R. W., Taylor, J. M., *J. Clin. Invest*., 76, 1501-1513, 1985; Weisgraber, K. H., Roses, A. D., Strittmatter, J., *Curr. Opin. Lipid.,* 5, 110-116, 1994).

Zusammenfassend kann festgestellt werden, daß in Bereichen des Gehirns und der Blut-Hirn-Schranke verschiedene Rezeptor-Systeme existieren, die einen Transport von ApoE bzw. ApoE-haltigen Partikeln ins Gehirn ermöglichen könnten.

Um die Rolle des Proteins nachzuweisen, war zu zeigen, daß eine Anreicherung eines Partikels in einem Gewebe erfolgt, nachdem Bindung des für die Anreicherung als ursächlich angesehenen Apolipoproteins E an die Oberfläche des Partikels vorgenommen wurde.

Für den Nachweis der Funktion von ApoE wurden Partikel eingesetzt, an deren Oberfläche ApoE aus Plasma nicht adsorbiert.

Verwendet wurden daher unmodifizierte Polybutylcyanoacrylat-(PBCA)-Partikel aus Beispiel 1, für welche die Abwesenheit von ApoE auf der Oberfläche nach Inkubation mit Plasma nachgewiesen wurde. Inkubation der Partikel mit einer Lösung von ApoE führte zu einer Adsorption auf der Oberfläche (Beispiel 2). Es ist somit möglich ApoE durch einen einfachen Adsorptionsprozeß an die Oberflächen partikulärer Träger zu binden.

### Überraschend ist mit ApoE somit ein Protein identifiziert worden, das gleichzeitig

a) in relativ großer Menge auf gewebespezifischen Partikeln vorhanden war,
b) bei dessen Anwesenheit eine ZNS-Wirksamkeit auftrat (Beispiel 1),
c) bei dessen Abwesenheit eine ZNS-Wirksamkeit fehlte (Beispiel 4),
d) und mit dem aufgrund der in der Literatur beschriebenen Rezeptoren ein Erkennungs- und Vermittlungseffekt auch theoretisch möglich ist.

Die richtige Konformation des adsorbierten Erkennungsproteins (an den Rezeptor bindendes Molekülsegment in der zum Rezeptor passenden Molekülanordnung exponiert) ist von essentieller Bedeutung für den Transport zum Zielgewebe, hier das ZNS. Unter Zulassungsgesichtspunkten für Arzneimittel (Vermeidung einer "new entity") sollte das Erkennungsprotein - wenn möglich - an die Partikel nicht kovalent gebunden werden, da dies für jede Arzneiform bei der Zulassung eine neue Toxizitätsstudie erforderlich macht. Technologisch einfacher ist die Bindung durch Adsorption an die Oberfläche.

Probleme dabei sind:
1. Adsorbiert das ApoE an die Partikeloberfläche auch ohne Gegenwart eines Tensids wie z.B. Tween® 80, welches zur automatischen Anreicherung von ApoE führt?
2. Verbleibt ApoE bei Bindung durch Adsorption nach intravenöser Injektion auf der Partikeloberfläche?
3. Ist ApoE in der richtigen Konformation adsorbiert, so daß es an das Zielgewebe binden kann und eine ZNS-Wirksamkeit vermittelt?

Besonders die mögliche Verdrängung von ApoE von der Oberfläche (siehe 2.) und die Notwendigkeit der richtigen Konformation des adsorbierten ApoE (siehe 3.) ließen es unwahrscheinlich erscheinen, daß ein Arzneistofftransport zum Gehirn erfolgen würde.

Präadsorbiertes ApoE könnte nach einer intravenösen Applikation möglicherweise von anderen Plasmaproteinen von der Partikeloberfläche verdrängt werden. Um diesen möglichen Verdrängungsprozeß zu simulieren und zu analysieren, wurden die Plasmaproteinadsorptionsmuster von PBCA-Partikeln bestimmt, an deren Oberfläche zuvor ApoE durch Adsorption gebunden worden war. Präadsorbiertes ApoE blieb auch nach nachträglicher Inkubation in Plasma auf der Oberfläche erhalten (Beispiel 3).

Der beobachtete analgetische Effekt resultiert aus einem Rezeptor-vermittelten Prozeß an der Blut-Hirn-Schranke. Daher muß auf der Oberfläche adsorbiertes ApoE in einer Konformation vorliegen, die eine Bindung an einen ApoE-Rezeptor zuläßt. Dies gilt für präadsorbiertes oder kovalent gebundenes ApoE ebenso wie für für ApoE-Moleküle, die durch Adsorption aus Plasma auf die Partikeloberfläche gelangen.

Tensidfreie PBCA-Partikel mit präadsorbiertem ApoE wurden im "tail-flick-Test" an Mäusen hinsichtlich ihres Potentials untersucht, Dalargin durch die Blut-Hirn-Schranke zu transportieren und einen analgetischen Effekt hervorzurufen. Unmodifizierte PBCA-Partikel waren nicht in der Lage, nach i.v. Applikation die Blut-Hirn-Schranke zu überwinden (Alyautdin, R. N., Gothier, D., Petrov, V. E., Kharkevich, D. A., Kreuter, J., *Eur. J. Pharm. Biopharm*., 41, 44-48, 1995; Kreuter, J., Alyautdin, R. A., Kharkevich, D. A., Ivanov, A., *Brain Res*., 674, 171-174, 1995; Schröder, U., Sabel, B. A., *Brain Res.,* 710, 121-124, 1996; Alyautdin, R. N., Petrov, V. E., Langer, K., Berthold, A., Kharkevich, D. A., Kreuter, J., *Pharm. Res.,* 14, 325-328, 1997). Die i.v. Applikation der PBCA-Nanopartikel mit präadsorbiertem ApoE führte jedoch zu einer analgetische Wirkung. 15 Minuten nach Injektion der Partikel wurde ein Effekt beobachtet, der 44% des maximal möglichen Effektes ausmachte (Beispiel 1). Entgegen den Erwartungen adsorbierte ApoE in der richtigen Konformation und blieb auch nach i.v. Injektion ausreichend stabil auf der Oberfläche adsorbiert, um Darlagin in das ZNS zu transportieren.

Die Tatsache, daß unmodifizierte Partikel, an die vor der i.v. Applikation ApoE gebunden wurde, einen analgetischen Effekt erzeugen, belegt die Beteiligung von ApoE an dem erhöhten Transport der Dalargin-beladenen PBCA-Partikel durch die Blut-Hirn-Schranke.

Für ApoE wird ein Modell beschrieben, nach dem das Protein aus zwei strukturell verschiedenen Domänen besteht (Weisgraber, K. H., *Adv. Prot. Chem*., 45, 249-303, 1994; Weisgraber, K. H., Roses, A. D., Strittmatter, J., *Curr. Opin. Lipid.,* 5, 110-116, 1994). Demnach besitzt ApoE eine amino-terminale globuläre (d.h. strukturell stabile) Domäne und eine carboxy-terminale labilere Domäne, die stärker zur Entfaltung ihrer Struktur neigt. Die N-terminale Domäne enthält den Teil des Proteins, der mit potentiellen Rezeptoren interagiert, die C-terminale Domäne ist für die Bindung an Lipide (z.B. in HDL) verantwortlich (Weisgraber, K. H., *Adv. Prot. Chem.,* 45, 249-303, 1994). Überraschenderweise reichte die Affinität dieser C-terminalen Domäne zu Oberflächen aus, daß
a) eine ausreichende Bindung von ApoE an die Partikeloberfläche stattfand,
b) die Adsorption ausreichend stabil war um ApoE auch bei Kontakt mit Plasmaproteinen auf der Oberfläche präsent zu halten,
c) die C-terminale Domäne und nicht die N-terminale Domäne auf der Oberfläche band und
d) dadurch die Struktur der stabileren N-terminalen Domäne und damit die Affinität zu potentiellen Rezeptoren erhalten blieb.

### Überraschend ist mit ApoE somit ein Protein identifiziert worden, das gleichzeitig

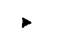 an die Oberfläche eines Partikels adsorbiert,
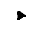 an die Oberfläche stabil gebunden bleibt und nicht vollständig durch andere Plasmaproteine verdrängt wird (z.B. nach intravenöser Injektion) und
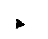 in der richtigen Konformation adsorbiert, so daß es an das Zielgewebe (in diesem Fall Blut-Hirn-Schranke und ZNS) binden kann und eine ZNS-Wirksamkeit des Arzneistoffes vermittelt.

Das oder die Erkennungsproteine können auf die Oberfläche unmodifizierter Partikel adsorbiert werden (Beispiel 2, PBCA-Partikel) oder alternativ nach vorheriger Modifikation der Oberfläche (Beispiel 2, PBCA-Partikel modifiziert durch adsorbiertes Tensid). Für die gewebespezifische anreicherung im Gehirn müssen dafür gezielt Tenside ausgewählt werden, die beispielsweise ApoE an der Oberfläche anreichern (z.B. Tween, Beispiel 2), damit in vivo eine ZNS-Wirkung auftritt (Tabelle 1). Tenside, die dies nicht bewirken, wie Poloxamer 407 (Beispiel 4) ergeben in vivo auch keine Arzneistoffwirkung im ZNS (Tabelle 1).

Die Adsorption der Erkennungsproteine kann dabei ex vivo erfolgen (z.B. aus ApoE-Lösung, Plasma, Serum, Blut), um in vivo eine ZNS-Wirkung zu erzeugen (Beispiel 1). Nach gezielter Modifikation der Partikeloberfläche zur präferentiellen Adsorption von ApoE kann dies jedoch auch in vivo nach Kontakt mit Blut erfolgen (Tabelle 1).

Bei Arzneistoffträgern mit sehr hydrophiler Oberfläche kann es sein, daß die Affinität des Erkennungsproteins nicht ausreicht, um an die Oberfläche im ausreichenden Maß zu adsorbieren. Ebenso ist es möglich, daß das zur Bindung an den Rezeptor benötigte Molekülsegment adsorbiert (im Fall von ApoE z.B. die N-terminale Domäne). In diesen Fall können die Erkennungsproteine an funktionelle Gruppen der Oberfläche kovalent gebunden werden, wobei die Bindung an die Oberfläche über Molekülsegmente erfolgt, die für die Bindung an den Rezeptor des Zielgewebes nicht benötigt werden (bei ApoE z.B. die C-terminale Domäne). Alternativ zur Anbindung des gesamten Erkennungsproteins können auch Teile des Moleküls verwandt werden, die den an den Rezeptor bindnenden Molekülteil enthalten.

In nachfolgender Tabelle sind Beispiele von relevanten Aktivierungs- und Kopplungsreagentien für eine chemisch-kovalente Kopplung des Proteins an die Trägermatrix aufgelistet.

Folgende Substanzen werden für die chemische Aktivierung, als Vorstufe einer kovalenten Verknüpfung von funktionellen Gruppen eines Biomoleküls mit denen auf einem festen Träger, eingesetzt.

| Partikel | Biomolckül | Substanz * |
|---|---|---|
| -COOH | -NH₂ | Carbodiimid (wasserlöslich) (1-Ethyl-3-(3-dimethylaminopropyl)-carbodiimidhydrochlorid) |
| | | |
| -COOH | -NH₂ | EEDQ (N-Ethoxycarbonyl-2-ethoxy-1,2-dihydrochinolin) |
| | | |
| -NH₂ | -NH₂ | Glulardialdehyd |
| | | |
| -OH (in aq.) | -NH₂ | Bromzyan (BrOCN) |
| | | |
| -CH₂-CH₂-OH | -NH₂ | meta-Perjodat (Na/K) JO₄⁻ |
| | | |
| -OH (in org.LM) | -NH₂ | Tosylchlorid |
| | | |
| -OH (in org.LM) | -NH₂ | Chlorameisensäureester |

| Sogenannte "ready to use" - Partikel mit reakliven Oberflächengruppen benötigen keine chemische Aktivierung als Kopplungsvorstufe: | | |
|---|---|---|
| -CH(O)-CH2 Epoxy- | -NH₂ -OH -COOH | ----- |
| | | |
| -CHO Aldehyd | -NH₂ | ----- |

Im allgemeinen können die Träger folgende chemische Wirkstoffgruppen enthalten:
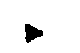 hydroxylierte Kohlenwasserstoffe
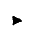 Carbonylverbindungen wie Ketone (z.B. Haloperidol), Monosaccharide, Disaccharide und Aminozucker
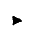 Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide (z.B. Ciclosporin), Polypeptide, β-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
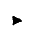 Kohlensäurederivate wie Urethane und Thiourethane, Harnstoff und Harnstoffderivate, Guanidinderivate, Hydantoine, Barbitursäurederivate und Thiobarbitursäurederivate
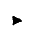 Nitroverbindungen wie aromatische Nitroverbindungen und heteroaromatische Nitroverbindungen
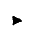 Amine wie aliphatische Amine, Aminoglykoside, Phenylalkylamine, Ephedrinderivate, Hydroxyphenylethanolamine, Adrenalinderivate, Amfetaminderivate, aromatische Amine und Derivate, quartäre Ammmoniumverbindungen
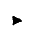 schwefelhaltige Verbindungen wie Thiole und Disulfane
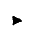 Sulfone, Sulfonsäureester und Sulfonsäureamide
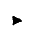 Polycarbocyclen wie Tetracycline, Steroide mit aromatischem Ring A, Steroide mit alpha,beta-ungesättigter Carbonylfunktion im Ring A und alpha Ketol-Gruppe (oder Methylketo-Gruppe) am C-17, Steroide mit einem Butenolid-Ring am C-17, Steroide mit einem Pentadienolid-Ring am C-17 und Seco-Steroide
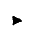 0-haltige Heterocyclen wie Chromanderivate (z.B. Cromoglicinsäure)
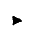 N-haltige Heterocyclen wie Pyrazolderivate (z.B. Propyphenazon, Phenylbutazon)
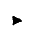 Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbanderivate, Pyrrolidinderivate, Purinderivate (z.B. Allopurinol), Xanthinderivate, 8-Hydroxychinolin-derivate, Amino-hydroxy-alkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
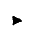 S-haltige Heterocyclen wie Thioxanthenderivate (z.B Chlorprothixen)
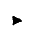 N,O- und N,S-haltige Heterocyclen wie monocyclische N,O-haltige Heterocyclen, monocyclische N,S-haltige Heterocyclen, Thiadiazinderivate, bicyclische N,S-haltige Heterocyclen, Benzothiadiazinderivate, tricyclische N,S-haltige Heterocyclen und Phenothiazinderivate
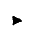 O,P,N-haltige Heterocyclen (z.B. Cyclophosphamid).

Beispiele für speziell in die Träger einzuarbeitende Arzneistoffgruppen und Arzneistoffe (als Salz, Ester, Ether oder in freier Form) sind:
Analgetika/Antirheumatika
   BTM Basen wie Morphin, Codein, Heroin, Piritamid, Diamorphin, Dihydrocodein, Hydromorphon, Hydrocodon, Pethidin, Fenpipramid, Piritramid, Clofedanol, Pentazocin, Buprenorphin, Nalbuphin, Tilidin, Fentanyl und Fentanylderivate, Levomethadon, Tramadol, Diclofenac, Ibuprofen, Indometacin, Naproxen, Piroxicam, Penicillamin, Ademetionin , Flupirtin, Acetylsalicylsäure
Antiallergika
   Pheniramin, Dimetinden, Terfenadin, Astemizol, Loratidin, Doxylamin, Meclozin, Bamipin, Clemastin
Antiasthmatika
   Terbutalin, Beclomethason, Cromoglycinsäure, Reproterol, Salbutamol, Nedocromil
Antibiotika/Chemotherapeutika
   hiervon: Rifampicin, Amoxicillin, Azlocillin, Bacampicillin, Benzylpenicillin, Amikacin, Azithromycin, Ciprofloxacin, Norfloxacin, Polypeptidantibiotika wie Colistin, Polymyxin B, Teicplanin, Vancomycin; Malariamittel wie Chinin, Halofantrin, Mefloquin, Chloroquin, Virustatika wie Ganciclovir, Foscarnet, Zidovudin, Aciclovir und andere wie Brivudin, Dapson, Fosfomycin, Fusafungin, Trimetoprim, Amphotericin
Antidota
   Mesna
Antiemetka
   Tropisetron, Scopolamin, Thiethylperazin
Antiepileptika
   Phenytoin, Mesuximid, Ethosuximid, Primidon, Phenobarbital, Valproinsäure, Carbamazepin, Clonazepam, Diazepam, Nitrazepam, Vigabatrin, Lamotrigin, Trimethadion, Sultiam
Antifibrinolytika
   Aminomethylbenzoesäure
Antihypertonika/Betarezeptorenblocker/Calciumantagonisten/ACE-Hemmer
   Bupranolol, Captopril, Fosinopril, Nitroprussidnatrium, Isradipin, Mepindolol
Antihypotonika
   Cafedrin, Dihydroergotamin
Antikoagulantien
   Heparin, Certoparin
Antimykotika
   Nystatin, Natamycin, Amphotericin B, Flucytosin, Miconazol, Fluconazol, Itraconazol, Clotrimazol, Econazol, Tioconazol, Fenticonazol, Bifonazol, Oxiconazol, Ketoconazol, Isoconazol, Tolnaftat, Amorolfin, Terbinafin
Corticoide
   Aldosteron, Fludrocortison, Betametason, Dexametason, Triamcinolon, Fluocortolon, Hydroxycortison, Prednisolon, Prednyliden, Cloprednol, Methylpredinsolon
Diagnostika
   a) radioaktive Isotope wie Te99m, In111 oder I131, kovalent gebunden an Lipide oder Lipoide oder andere Moleküle oder in Komplexen
   b) hochsubstituiertre iodhaltige Verbindungen wie z.B. Lipide
   c) Megluminamidotrizoat, Iotroxinsäure, Natriumiopodat
Diuretika
   Hydrochlorothiazid
Erythropoetin
Fibrinolytika
   Urokinase
Hämostyptika/Antihämorrhagika
   Blutgerinnungsfaktoren VIII, IX
Hypnotika, Sedativa
   Cyclobarbital, Pentobarbital, Phenobarbital, Methaqualon (BTM), Benzodiazepine (Flurazepam, Midazolam, Nitrazepam, Lormetazepam, Flunitrazepam, Triazolam, Brotizolam, Temazepam, Loprazolam), Thalidomid, Zolpidem, Zopiclon, Diphenhydramin, Doxylamin, Temazepam
Hypophysen-, Hypothalamushormone, regulatorische Peptide und ihre Hemmstoffe
   Corticotrophin, Tetracosactid, Choriongonadotropin, Urofollitropin, Urogonadotropin, Somatropin, Metergolin, Bromocriptin, Terlipressin, Desmopressin, Oxytocin, Argipressin, Ornipressin, Leuprorelin, Triptorelin, Gonadorelin, Buserelin, Nafarelin, Goselerin, Somatostatin, Quinagolid, Octreotidacetat, Lypressin
Immuntherapeutika und Zytokine
   Dimepranol-4-acetatamidobenzoat, Thymopentin, a-Interferon, β-Interferon, g-Interferon, Filgrastim, Interleukine, Azathioprin, Ciclosporin, Molgramostim, GM-CSF
Koronarmittel
   Glyceroltrinitrat, Isosorbiddinitrat, Oxyfedrin
Lebertherapeutika
   Sylimarin
Lipidsenkert
   Pravaststin, Fluvastatin
Lokalanaesthetika
   Butanilicain, Mepivacain, Bupivacain, Etidocain, Lidocain, Articain, Prilocain, Propipocain, Oxybuprocain, Tetracain, Benzocain
Migränemittel
   Proxibarbal, Lisurid, Methysergid, Dihydroergotamin, Clonidin, Ergotamin, Pizotifen, Sumatriptan
Muskelrelaxantien
   Tubocurarin, Alcuronium, Pancuronium, Vecuronium, Atracurium, Suxamethonium, Dantrolen, Baclofen, Carisoprodol, Chlormezanon, Memantin, Tizanidin
Narkosemittel
   Methohexital, Propofol, Etomidat, Ketamin, Alfentanil, Thiopental, Droperidol, Fentanyl, Alfentanil, Sufentanil
Nebenschilddrüsenhormone, Calciumstoffwechselregulatoren
   Dihydrotachysterol, Calcitonin, Clodronsäure, Etidronsäure, Pamidronsäure
Neuropathiepräparate
   α-Liponsäure
Prostaglandine
   Alprostadil
Psychopharmaka
   Benzodiazepine (Lorazepam, Diazepam), Clomethiazol,
Schilddrüsentherapeutika
   1-Thyroxin, Carbimazol, Thiamazol, Propylthiouracil
Sera, Immungiobuline, Impfstoffe
   a) Immunglobuline allgemein und spezifisch wie Hepatitis-Typen, Röteln, Cytomegalie, Tollwut, FSME, Varicella-Zoster, Tetanus, Rhesusfaktoren
   b) Immunsera wie Botulismus-Antitoxin, Diphterie, Gasbrand, Schlangengift, Skorpiongift
   c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus, Tollwut, Typhus
Sexualhormone und ihre Hemmstoffe
   Anabolika, Androgene, Antiandrogene, Gestagene, Estrogene, Antiestrogene (Tamoxifen etc.), Flutamid, Fosfestrol, Cyproteron, Formestan, Aminoglutethimid
Toxoplasmosemittel
   Atovaquon
Urologika
   Trospiumchlorid
Vitamine
   Alfacalcidol, Vitamin A und -derivate, Vitamin E und -derivate, Ascorbinsäure
ZNS-Therapeutika
   a) Neuroleptika wie Perazin, Promazin, Sulpirid, Thioridazin, Chlorprothixen, Levomepromazin, Prothipendyl, Chlorpromazin, Clopenthixol, Triflupromazin, Perphenazin, Trifluperazin, Pimozid, Reserpin, Fluphenazin, Haloperidol, Trifluperidol, Benperidol, Alimemazin, Fluphenazin, Flupentixol, Melperon, Bromperidol, Pipamperon, Clozapin, Risperidon,
   b) Antidepressiva wie Imipramin, Desipramin, Trimipramin, Lofepramin, Clomipramin, Opipramol, Amitriptylin, Amitriptylinoxid, Nortriptylin, Dibenzepin, Doxepin, Maprotilin, Mianserin, Fluoxetin, Fluvoxamin, Paroxetin, Trazodon, Moclobemid, Tranylcypromin, Oxitriptan, Viloxazin, Hypericin, Lithiumsalze
   c) Tranquillantien wie Meprobamat, Hydroxyzin, Benzodiazepine wie Chlordiazepoxid, Diazepam, Prazepam, Oxazepam, Dikalium-clorazepat, Lorazepam, Clonazepam, Bromazepam, Clotiazepam, Alprazolam, Clobazam, Buspiron
   d) Psychostimulantien wie Coffein, Theophyllin, Theobromin, Amphetamine und verwandte Substantzen
   e) Stoffe zur Behandlung dementieller Syndrome wie Meclofenoxat, Nicergolin, Piracetam, Pyritinol, Tacrin, Memantin, Dihydroergotoxinmethansulfonat
   f) Appetitzügler wie Nor-pseudoephedrin, Amfepramon, Mefenorex, Levopropylhexedrin, Fenfluramin, Dexfenfluramin
   g) Analeptika wie Doxapram, Fenetyllin
   h) Nervenwachstumsfaktor (Nerve growth factor), Naloxon, Dalargin
   i) Antiparkinsonmittel wie L-Dopa, Selegilin, Bromocriptin, Amantadin, Tiaprid, Biperiden, Trihexylphenidyl, Procyclidin, Benzatropin, Orphenadrin, Bornaprin, Metixen, α-Dihydroergocryptin, Carbidopa
Zystostatika und Metastasenhemmer
   a) Alkylantien wie Nimustin, Melphalan, Carmustin, Lomustin, Cyclophosphamid, Ifosfamid, Trofosfamid, Chlorambucil, Busulfan, Treosulfan, Prednimustin, Thiotepa,
   b) Antimetabolite wie Cytarabin, Fluorouracil, Methotrexat, Mercaptopurin, Tioguanin
   c) Alkaloide wie Vinblastin, Vincristin, Vindesin
   d) Antibiotika wie Aclarubicin, Bleomycin, Dactinomycin, Daunorubicin, Doxorubicin, Epirubicin, Idarubicin, Mitomycin, Plicamycin
   e) Komplexe von Nebengruppenelementen (z.B. Ti, Zr, V, Nb, Ta, Mo, W, Ru, Pt) wie Carboplatin, Cisplatin und Metallocenverbindungen wie Titanocendichlorid
   f) Amsacrin, Dacarbazin, Estramustin, Etoposid, Hydroxycarbamid, Mitoxanthron, Procarbazin, Temiposid
   g) Alkylamidophospholipide (beschrieben in J.M. Zeidler, F. Emling, W. Zimmermann und Roth, H. J., Archiv der Pharmazie, 324, 687, 1991)
   h) Etherlipide wie Hexadecylphosphocholin, Ilmofosin und Analoga, (beschrieben in Zeisig, R., Arndt, D., Brachwitz, H., Pharmazie 45 809-818 1990)
   i) Taxane wie Paclitaxel und Docetaxel
   j) Altretamin, Aminoglutethimid, Asparaginase, Hydroxycarbamid, Miltefosin

### Beispiele

### Beispiel 1:

Unmodifizierte, Dalargin-beladene PBCA-Nanopartikel waren nicht in der Lage, nach i.v. Applikation die Blut-Hirn-Schranke zu überwinden. Die i.v. Applikation dieser Nanopartikel mit präadsorbiertem ApoE an Mäuse führte jedoch in dem von Alyautdin et al. beschriebenen "tail-flick-Test" (Alyautdin, R. N., Gothier, D., Petrov, V. E., Kharkevich, D. A., Kreuter, J., *Eur. J. Pharm. Biopharm.,* 41, 44-48, 1995) zu einer analgetische Wirkung. 15 Minuten nach Injektion der Partikel wurde ein Effekt beobachtet, der 44% des maximal möglichen Effektes ausmachte (Berechnung des Effektes nach einer von Alyautdin et al. (Alyautdin, R. N., Gothier, D., Petrov, V. E., Kharkevich, D. A., Kreuter, J., *Eur. J. Pharm. Biopharm*., 41, 44-48, 1995) angegebenen Formel).

### Beispiel 2:

Es wurden ApoE-Lösungen (50 mg ApoE in 160 µl NH₄HCO₃-Puffer (10 mM), pH 7,5, Calbiochem-Novabiochem, Nottingham, UK) in jeweils 500 µl einer 6prozentigen Suspension (m/V) von unmodifizierten und mit Tween® 80 modifizierten PBCA-Partikeln für 3 h bei 37°C (Tamada und Ikada, 1993) inkubiert. Die Partikel wurden durch Zentrifugation vom Dispersionsmedium separiert und viermal gewaschen. Das adsorbierte ApoE wurde mit von Hochstrasser et al. (Hochstrasse, D. F., Harrington, M. G., Hochstrasser, A.-C., Miller, M. J., Merril, C. R., Anal. Biochem., 173, 424-435, 1988) beschriebenen solubilisierenden Lösungen von der Partikeloberfläche desorbiert. Jeweils 80 µl der proteinhaltigen Lösungen wurden auf die Röhrchengele der 1. Dimension der 2-DE appliziert. Der Nachweis des adsorbierten ApoE erfolgte mit zweidimensionaler Elektrophorese (2-DE) nach Blunk (Blunk, T., Hochstrasser, D. F., Sanchez, J.-C., Müller, B. W., Müller, R. H., *Electrophoresis,* 14, 1382-1387, 1994).

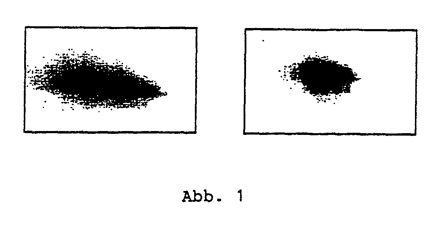

Abb. 1 zeigt die resultierenden ApoE-Spots auf den 2-DE-Gelen der unmodifizierten und mit Tween® 80 modifizierten PBCA-Partikel. Die Anreicherung von ApoE ist dabei auf den unmodifizierten Partileln am höchsten.

### Beispiel 3:

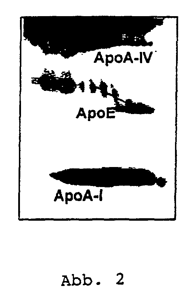

Um zu zeigen, daß andere Proteine das auf den PBCA-Partikeln präadsorbierte ApoE nach einer i.v. Applikation von der Partikeloberfläche nicht vollständig verdrängen, wurden die Partikel mit präadsorbiertem ApoE (vgl. Beispiel 2) gemäß dem Standardprotokoll für die 2-DE in Plasma inkubiert (5 min bei 37°C, nach Blunk, siehe Beispiel 2) und die resultierenden Adsorptionsmuster bestimmt. Abb. 2 zeigt Ausschnitte aus dem 2-DE-Gel, die die ApoE-Spots enthalten.

### Beispiel 4:

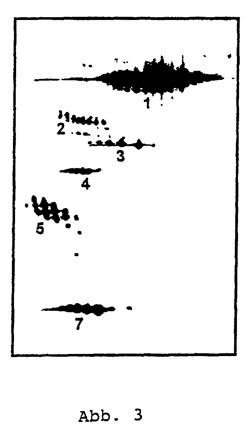

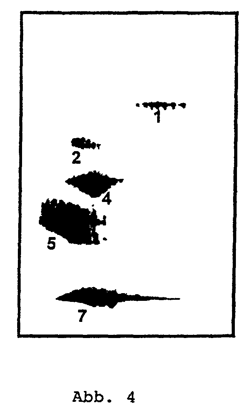

Es wurden die Plasmaproteinadsorptionsmuster von PBCA-Partikeln bestimmt, die keine analgetische Wirkung von Darlagin im ZNS vermittelten. Abb. 3 zeigt das mit unmodifizierten PBCA-Partikeln erhaltene Gel. Es wurde kein ApoE detektiert. Abb. 4 zeigt das mit Poloxamer 407 modifizierten PBCA-Partikeln erhaltene Gel. Es wurde ebenfalls kein ApoE detektiert und somit gezeigt, daß es in Abwesenheit von ApoE zu keiner ZNS-Wirksamkeit kommt.

**Tabelle 1:**

| Tabelle 1: ZNS-Wirksamkeit von unmodifizierten und mit verschiedenen Tensiden oberflächenmodifizierten, mit Darlagin beladenen PBCA-Nanopartikeln nach intravenöser Gabe an Mäuse (Dalargin-Dosis: 10 mg/kg). Angegeben sind die analgetischen Effekte ausgedrückt als Prozentsatz vom maximal möglichen Effekt (% MME) (S: Standardabweichung, n=4) (Berechnungsformel nach Alyautdin, R. N., Gothier, D., Petrov, V. E., Kharkevich, D. A., Kreuter, J., *Eur. J. Pharm. Biopharm.,* 41, 44-48, 1995) | | | | |
|---|---|---|---|---|
| oberflächen-modifizierendes Tensid | % MME (nach 15 min) | S | % MME (nach 45 min) | S |
| Tween 20 | 79,7 | ± 21,3 | 52,9 | ± 20,9 |
| Tween 40 | 87,5 | ± 16,1 | 60,8 | ± 38,0 |
| Tween 60* | -7,1 | ± 24,2 | 45,5 | ± 36,6 |
| Tween 80 | 100 | ± 0 | 10,5 | ± 14,9 |
| Poloxamer 407 | 4,4 | ± 3,9 | 9,5 | ± 5,8 |
| Poloxamer 908 | -1,3 | ± 3,6 | 4,2 | ± 5,5 |
| Poloxamer 188 | 8,1 | ± 5,9 | 3,3 | ± 3,4 |
| Poloxamer 184 | 0,9 | ± 0,28 | 1,0 | ± 2,3 |
| Poloxamer 338 | 0,2 | ± 0,5 | 1,4 | ± 3,9 |
| Cremophor EL | 10,9 | ± 13,1 | 8,6 | ± 8,7 |
| unmodifizierte Partikel | 2,3 | ± 1,6 | 3,7 | ± 11,7 |

| | | | | |
|---|---|---|---|---|
| *Dalargin-Dosis: 7,5mg/kg | | | | |

- **Abb. 1:**: Ausschnitte mit ApoE-Spots aus 2-DE-Gelen von unmodifizierten PBCA-Partikeln (links) und von mit Tween 80 modifizierten PBCA-Partikeln (rechts) jeweils nach Präadsorption von ApoE. Durch das adsorbierte ApoE besaßen auch die unmodifizierten Partikel eine ZNS-Wirksamkeit
(Beispiel 2).
- **Abb. 2:**: Ausschnitt aus dem 2-DE-Gel von unmodifizierten PBCA-Partikeln mit präadsorbiertem ApoE nach Inkubation in Plasma (Beispiel 3).
Abszisse: nicht linearer Gradient pI 4,5 - 6,0
Ordinate: nicht linearer Gradient MG 25.000 - 46.000
- **Abb. 3:**: 2-DE-Gel von unmodifizierten PBCA-Partikeln ohne ZNS-Wirksamkeit (Beispiel 4).
(1) Albumin, (2) α1-Antitrypsin, (3) Fibrinogen γ, (4) ApoA-IV, (5) ApoJ, (7) ApoA-I
Abszisse: nicht linearer Gradient pI 4,5 - 6,5
Ordinate: nicht linearer Gradient MG 25.000 - 75.000
- **Abb. 4:**: 2-DE-Gel von mit Poloxamer 407 modifizierten PBCA-Partikeln ohne ZNS-Wirksamkeit (Beispiel 4)
(1) Albumin, (2) α1-Antitrypsin, (4) ApoA-IV, (5) ApoJ, (7) ApoA-I
Abszisse: nicht linearer Gradient pI 4,5 - 6,5
Ordinate: nicht linearer Gradient MG 25.000 - 75.000

## Patentansprüche

1. Verwendung von Arzneistoffträgerpartikeln in Form von festen Teilchen oder Suspensionen fester Teilchen in Wirkstoff-beladener Form, wobei das Partikelmaterial Polybutylcyanoacrylat (PBCA) Polymer, den Arzneistoff in form von Nanosuspensionen bzw. Hydrosole oder feste Lipide umfaßt, an die Partikeloberfläche Apolipoprotein E oder zumindest ein Rezeptorerkennender Molekülteil davon gebunden ist, zur Herstellung von Arzneimitteln, die im zentralen Nervensystem oder an Zielorten oder in Zellen im Gehirn anreicherbar sind.

2. Verwendung nach Anspruch 1, **dadurch gekennzeichnet, daß** das Erkennungsprotein Apolipoprotein E in Kombination mit einem oder mehreren anderen Erkennungsproteinen vorliegt, insbesondere mit Apolipoprotein A-I und/oder A-IV.

3. Verwendung nach Anspruch 1 oder 2, **dadurch gekennzeichnet, daß** Apo A-I und/oder Apo A-IV einzeln oder in Kombinationen eingesetzt werden.

4. Verwendung nach einem der Ansprüche 1 bis 3, **dadurch gekennzeichnet, daß** das Apo E, gegebenenfalls in Kombination mit anderem Erkennungsprotein, bezogen auf den Arzneistoffträger, in einer Menge von 0,001 bis 40 Gew.-%, insbesondere von 0,01 bis 30 Gew.-% und bevorzugt von 0,1 bis 15 Gew.-% vorhanden ist.

5. Verwendung nach einem der Ansprüche 1 bis 4, **dadurch gekennzeichnet, daß** die Arzneistoffträgerpartikel in Form von festen Teilchen oder Suspensionen fester Teilchen vorliegen, die vorzugsweise amorphe oder kristalline Nanooder Mikropartikel umfassen.

6. Verwendung nach einem der Ansprüche 1 bis 5, **dadurch gekennzeichnet, daß** das Apo E, gegebenenfalls in Kombination mit anderem Erkennungsprotein kovalent oder durch unspezifische oder spezifische Adsorption, insbesondere durch präferentielle Adsorption, an die Oberfläche der Partikel gebunden ist.

7. Verwendung nach einem der Ansprüche 1 bis 6, **dadurch gekennzeichnet, daß** die Oberfläche der Partikel vor der präferentiellen Adsorption durch Einführung funktioneller Gruppen chemisch modifiziert wurde oder durch physikalische Behandlung mit Plasma in den Eigenschaften verändert wird.

8. Verwendung nach Anspruch 6, **dadurch gekennzeichnet, daß** die kovalente Bindung an Partikel mit Epoxy- oder Aldehydgruppen oder nach Aktivierung der Partikeloberfläche mit Aktivatoren, insbesondere Carbodiimid, N-Ethoxycarbonyl-2-ethoxy-1,2-dihydrochinolin, Glutardialdehyd, Bromzyan, meta-Perjodat (Na-Salz oder K-Salz), Tosylchlorid und Chlorameisensäureester, bewirkt wird.

9. Verwendung nach Anspruch 8, **dadurch gekennzeichnet, daß** die Bindung des Apo E, gegebenenfalls in Kombination mit anderem Erkennungsprotein, über ihre Aminogruppen bewirkt wird.

10. Verwendung nach Anspruch 6, **dadurch gekennzeichnet, daß** die präferentielle Adsorption aus Proteinlösungen oder durch Kontakt der Partikel mit Plasma, Serum oder Blut ex vivo erfolgt.

11. Verwendung nach Anspruch 6 oder 10, **dadurch gekennzeichnet, daß** die Oberfläche der Partikel vor der präferentiellen Adsorption durch Einführung von Hydroxyl-, Carboxyl-, Amino-, Hydroxyethyl-, Epoxy- oder Aldehydgruppen und deren Derivate chemisch modifiziert wurde oder durch plasma etching zur Einführung von Hydroxylgruppen in den Eigenschaften verändert wird.

12. Verwendung nach Anspruch 6, 10 oder 11, **dadurch gekennzeichnet, daß** die Oberfläche durch Adsorption von Substanzen modifiziert wird, die zu einer präferentiellen Adsorption des Apo E, gegebenfalls in Kombination mit anderem Erkennungsprotein, führen, wobei die modifizierende Substanz im Verhältnis zum Arzneistoffpartikel in einer gewichtsbezogenen Menge von 0,01 Teile bis 10 modifizierende Susbstanz pro 1 Teil Partikel, vorzugsweise 0,1 bis 10 Teile modifizierende Susbstanz pro 1 Teil Partikel, und insbesondere 1 Teil modifizierende Susbstanz pro 1 Teil Partikel eingesetzt wird.

13. Verwendung nach Anspruch 12, **dadurch gekennzeichnet, daß** die Oberfläche durch die Adsorption von Tensiden, insbesondere ethoxylierten Tensiden, vorzugsweise Polyethylenglykol-Fett-säureestern und Polyethylenglykol-Fettalkoholethern, bevorzugt Polyetylenglykol-Sorbitanfettsäureestern und Polyethylenglykol-Fettsäureglyceriden, bevorzugter Tween® 20, 40, 60 und 80 oder Cremophor® EL und RH40 modifiziert wird.

14. Verwendung nach Anspruch 12, **dadurch gekennzeichnet, daß** die Oberfläche durch die Adsorption von Polymeren, insbesondere Polymeren aus der Poloxameren und Poloxaminen, Cellulosen und ihren Derivate, vorzugsweise Methylcellulose, Hydroxyethylcellulose, Hydroxypropyl-Methylcellulose, Hydroxypropylcellulose, Carboxymethylcellulose-Natrium sowie Xanthan, Polyvinylalkohol, Polyvinylpyrrolidon, Polyacrylsäuren, Polyethylenglykolen, Polyethylenglykolenthaltenden Block-Copolymere, Stärke und -derivaten, Dextran und -derivaten, Polyethylenimin und Gelatine modifiziert wird.

15. Arzneistoffträgerpartikel in Form von festen Teilchen oder Suspensionen fester Teilchen in Wirkstoff-beladener Form, wobei das Partikelmaterial Poly-butylcyanoacrylat (PBCA) Polymer den Arzneistoff in form von Nanosuspensionen bzw. Hydrosolen oder feste Lipide umfaßt, an die Partikeloberfläche Apolipoprotein E durch unspezifische oder spezifische Adsorption oder kovalent an die Oberfläche der Partikel gebunden ist, wobei die kovalente Bindung an die Partikel mit reaktiven Oberflächengruppen bewirkt worden ist.

16. Arzneistoffträgerpartikel nach Anspruch 15, **dadurch gekennzeichnet, daß** die Bindung an Partikel mit Epoxy- oder Aldehydgruppen oder nach Aktivierung der Partikeloberfläche mit Aktivatoren, insbesondere Carbodiimid, N-Ethoxycarbonyl-2-ethoxy-1,2-dihydrochinolin, Glutardialdehyd, Bromzyan, meta-Perjodat (Na-Salz oder K-Salz), Tosylchlorid und Chlorameisensäureester, bewirkt worden ist.

17. Arzneistoffträgerpartikel nach Anspruch 16, **dadurch gekennzeichnet, daß** die Bindung des Apo E über seine Aminogruppen bewirkt worden ist.

18. Arzneistoffträgerpartikel nach einem der Ansprüche 15 bis 17, **dadurch gekennzeichnet, daß** das Apolipoprotein E in Kombination mit einem oder mehreren anderen Erkennungsproteinen vorliegt, insbesondere mit Apolipoprotein A-I und/oder A-IV.

19. Arzneistoffträgerpartikel nach einem der Ansprüche 15 bis 18, **dadurch gekennzeichnet, daß** Apo A-I und Apo A-IV einzeln oder in Kombinationen eingesetzt werden.

20. Arzneistoffträgerpartikel nach einem der Ansprüche 15 bis 19, **dadurch gekennzeichnet, daß** das Apo E, gegebenenfalls in Kombination mit anderem Erkennungsprotein, bezogen auf den Arzneistoffträger, in einer Menge von 0,001 bis 40 Gew.-%, insbesondere von 0,01 bis 30 Gew.-% und bevorzugt von 0,1 bis 15 Gew.-% vorhanden ist.

21. Arzneistoffträgerpartikel nach einem der Ansprüche 15 bis 20, **dadurch gekennzeichnet, daß** sie in Form von festen Teilchen oder von Suspensionen fester Teilchen vorliegen, die vorzugsweise amorphe oder kristalline Nano- oder Mikropartikel umfassen, oder von Nanosuspensionen oder Hydrosolen vorliegen.

## Claims

1. Use of medicament excipient particles in the form of solid particles or suspensions of solid particles in active-ingredient-loaded form, where the particulate material comprises polybutyl cyanoacrylate (PBCA) polymer, the medicament in the form of nanosuspensions or hydrosols or solid lipids, and apolipoprotein E or at least one receptor-recognizing moiety thereof is bonded to the particle surface, for the preparation of medicaments which can be concentrated in the central nervous system or at target sites or in cells in the brain.

2. Use according to Claim 1, **characterized in that** the recognition protein apolipoprotein E is present in combination with one or more other recognition proteins, in particular with apolipoprotein A-I and/or A-IV.

3. Use according to Claim 1 or 2, **characterized in that** apo A-I and/or apo A-IV are employed individually or in combinations.

4. Use according to one of Claims 1 to 3, **characterized in that** the apo E, if desired in combination with another recognition protein, is present in an amount of from 0.001 to 40% by weight, in particular from 0.01 to 30% by weight and preferably from 0.1 to 15% by weight, based on the medicament excipient.

5. Use according to one of Claims 1 to 4, **characterized in that** the medicament excipient particles are in the form of solid particles or suspensions of solid particles which preferably comprise amorphous or crystalline nanoparticles or microparticles.

6. Use according to one of Claims 1 to 5, **characterized in that** the apo E, if desired in combination with another recognition protein, is bonded to the surface of the particles covalently or through non-specific or specific adsorption, in particular through preferential adsorption.

7. Use according to one of Claims 1 to 6, **characterized in that** the surface of the particles has been chemically modified before the preferential adsorption through the introduction of functional groups or its properties are changed through physical treatment with plasma.

8. Use according to Claim 6, **characterized in that** the covalent bonding is effected to particles containing epoxide or aldehyde groups or after activation of the particle surface by means of activators, in particular carbodiimide, N-ethoxy-carbonyl-2-ethoxy-1,2-dihydroquinoline, glutaraldehyde, cyanogen bromide, meta-periodate (Na salt or K salt), tosyl chloride or chloroformic acid esters.

9. Use according to Claim 8, **characterized in that** the bonding of the apo E, if desired in combination with another recognition protein, is effected via its amino groups.

10. Use according to Claim 6, **characterized in that** the preferential adsorption is carried out ex vivo from protein solutions or through contact of the particles with plasma, serum or blood.

11. Use according to Claim 6 or 10, **characterized in that** the surface of the particles has been chemically modified before the preferential adsorption through the introduction of hydroxyl, carboxyl, amino, hydroxyethyl, epoxide or aldehyde groups or derivatives thereof or its properties are changed by plasma etching for the introduction of hydroxyl groups.

12. Use according to Claim 6, 10 or 11, **characterized in that** the surface is modified by adsorption of substances which result in preferential adsorption of the apo E, if desired in combination with another recognition protein, where the modifying substance is employed in an amount by weight relative to the medicament particle of from 0.01 to 10 parts of modifying substance per part of particles, preferably from 0.1 to 10 parts of modifying substance per part of particles, and in particular 1 part of modifying substance per part of particles.

13. Use according to Claim 12, **characterized in that** the surface is modified through the adsorption of surfactants, in particular ethoxylated surfactants, preferably polyethylene glycol fatty acid esters and polyethylene glycol fatty alcohol ethers, preferably polyethylene glycol sorbitan fatty acid esters and polyethylene glycol fatty acid glycerides, preferably Tween® 20, 40, 60 or 80 or Cremophor® EL or RH40.

14. Use according to Claim 12, **characterized in that** the surface is modified through the adsorption of polymers, in particular polymers from the poloxamers and poloxamines, celluloses and derivatives thereof, preferably methylcellulose, hydroxyethylcellulose, hydroxypropylmethylcellulose, hydroxypropylcellulose, carboxymethylcellulose sodium and xanthan, polyvinyl alcohol, polyvinylpyrrolidone, polyacrylic acids, polyethylene glycols, polyethylene glycol-containing block copolymers, starch and derivatives thereof, dextran and derivatives thereof, polyethylenimine and gelatine.

15. Medicament excipient particles in the form of solid particles or suspensions of solid particles in active-ingredient-loaded form, where the particulate material comprises polybutyl cyanoacrylate (PBCA) polymer, the medicament in the form of nanosuspensions or hydrosols or solid lipids, and apolipoprotein E is bonded to the particle surface through non-specific or specific adsorption or covalently bonded to the surface of the particles, where the covalent bonding to the particles has been effected by means of reactive surface groups.

16. Medicament excipient particles according to Claim 15, **characterized in that** the bonding has been effected to particles containing epoxide or aldehyde groups or after activation of the particle surface by means of activators, in particular carbodiimide, N-ethoxycarbonyl-2-ethoxy-1,2-dihydroquinoline, glutaraldehyde, cyanogen bromide, meta-periodate (Na salt or K salt), tosyl chloride or chloroformic acid esters.

17. Medicament excipient particles according to Claim 16, **characterized in that** the bonding of the apo E has been effected via its amino groups.

18. Medicament excipient particles according to one of Claims 15 to 17, **characterized in that** the apolipoprotein E is present in combination with one or more other recognition proteins, in particular with apolipoprotein A-I and/or A-IV.

19. Medicament excipient particles according to one of Claims 15 to 18, **characterized in that** apo A-I and apo A-IV are employed individually or in combinations.

20. Medicament excipient particles according to one of Claims 15 to 19, **characterized in that** the apo E, if desired in combination with another recognition protein, is present in an amount of from 0.001 to 40% by weight, in particular from 0.01 to 30% by weight and preferably from 0.1 to 15% by weight, based on the medicament excipient.

21. Medicament excipient particles according to one of Claims 15 to 20, **characterized in that** they are in the form of solid particles or suspensions of solid particles which preferably comprise amorphous or crystalline nanoparticles or microparticles, or of nanosuspensions or hydrosols.

## Revendications

1. Utilisation de particules d'excipient de médicament sous forme de particules solides ou de suspensions de particules solides sous forme chargée de substance active, dans lesquelles le matériau des particules comprend un polymère de polybutylcyanoacrylate (PBCA), le médicament sous forme de nanosuspensions, d'hydrosols ou de lipides solides, de l'apolipoprotéine E ou au moins une partie de molécule de l'apolipoprotéine E reconnaissant le récepteur est liée à la surface des particules, pour fabriquer des médicaments susceptibles d'être administrés au système nerveux central ou à des lieux ciblés ou dans les cellules du cerveau.

2. Utilisation selon la revendication 1, **caractérisée en ce que** la protéine de reconnaissance apolipoprotéine E est présente en association avec une ou plusieurs autres protéines de reconnaissance, en particulier avec l'apolipoprotéine A-I et/ou A-IV.

3. Utilisation selon la revendication 1 ou la revendication 2, **caractérisée en ce que** l'apo A-I et/ou l'apo A-IV ont utilisées seules ou en association.

4. Utilisation selon l'une des revendications 1 à 3, **caractérisée en ce que** l'apo E, le cas échéant en association avec une autre protéine de reconnaissance, par rapport à l'excipient de médicament, est présente en une quantité de 0,001 à 40 % en poids, en particulier de 0,01 à 30 % en poids et de préférence de 0,1 à 15 % en poids.

5. Utilisation selon l'une des revendications 1 à 4, **caractérisée en ce que** les particules d'excipient de médicament sont présentes sous forme de particules solides ou de suspensions de particules solides, qui comprennent de préférence des nanoparticules ou des microparticules amorphes ou cristallines.

6. Utilisation selon l'une des revendications 1 à 5, **caractérisée en ce que** l'apo E, le cas échéant en association avec une autre protéine de reconnaissance, est liée de manière covalente ou par adsorption non spécifique ou spécifique, en particulier par adsorption préférentielle, à la surface des particules.

7. Utilisation selon l'une des revendications 1 à 6, **caractérisée en ce que** la surface des particules a été modifiée chimiquement avant l'adsorption préférentielle par introduction de groupes fonctionnels ou modifiée dans ses propriétés par traitement physique avec du plasma.

8. Utilisation selon la revendication 6, **caractérisée en ce que** la liaison covalente aux particules est réalisée avec des groupes époxy ou aldéhyde ou après activation de la surface des particules par des activateurs, en particulier carbodiimide, N-éthoxycarbonyl-2-éthoxy-1,2-dihydroquinoline, glutardialdéhyde, bromure cyanique, métapreiodate (sel Na ou sel K), chlorure de tosyle et ester d'acide chloroformique.

9. Utilisation selon la revendication 8, **caractérisée en ce que** la liaison de l'apo E, le cas échéant en association avec une autre protéine de reconnaissance, a lieu par ses groupes amino.

10. Utilisation selon la revendication 6, **caractérisée en ce que** l'adsorption préférentielle à partir de solutions protéiques ou par contact des particules avec du plasma, du sérum ou du sang a lieu *ex vivo.*

11. Utilisation selon la revendication 6 ou 10, **caractérisée en ce que** la surface des particules est modifiée chimiquement avant adsorption préférentielle par introduction de groupes hydroxyle, carboxyle, amino, hydroxyéthyle, époxy ou aldéhyde et leurs dérivés ou est modifiée dans ces propriétés par mordançage au plasma pour introduction de groupes hydroxyle.

12. Utilisation selon la revendication 6, 10 ou 11, **caractérisée en ce que** la surface est modifiée par adsorption de substances qui entraînent une adsorption préférentielle de l'apo E, le cas échéant en association avec une autre protéine de reconnaissance, laquelle substance modifiée en relation avec la particule de médicament est utilisée en une quantité en relation avec le poids de 0,01 part à 10 parts de substance modifiée pour une part de particule, de préférence de 0,1 à 10 parts de substance modifiée pour une part de particule et en particulier une part de substance modifiée pour une part de particule.

13. Utilisation selon la revendication 12, **caractérisée en ce que** la surface est modifiée par l'adsorption de tensides, en particulier de tensides éthoxylés, de préférence des esters d'acide gras de polyéthylène glycol et des éthers d'alcool gras de polyéthylène glycol, de préférence des esters d'acide gras de polyéthylène glycol-sorbitane et des glycérides d'acide gras de polyéthylène glycol, de préférence Tween® 20, 40, 60 et 80 ou Cremophor® LE et RH40.

14. Utilisation selon la revendication 12, **caractérisée en ce que** la surface est modifiée par l'adsorption de polymères, en particulier des polymères appartenant aux poloxamères et au poloxamines, à la cellulose et à ses dérivés, de préférence la méthylcellulose, l'hydroxyéthylcellulose, l'hydroxypropylméthylcellulose, l'hydroxypropylcellulose, la carboxyméthylcellulose sodique ainsi que le xanthane, l'alcool polyvinylique, la polyvinylpyrrolidone, l'acide polyacrylique, les polyéthylène glycol, les copolymères blocs contenant du polyéthylène glycol, l'amidon et ses dérivés, le dextrane et ses dérivés, la polyéthylénimine et la gélatine.

15. Particules d'excipients de médicament sous forme de particules solides ou de suspensions de particules solides sous forme chargée de substance active, dans lesquelles le matériau des particules comprend un polymère de polybutylcyanoacrylate (PBCA), la substance médicamenteuse sous forme de nanosuspensions, d'hydrosole ou de lipides solides, l'apolipoprotéine E est liée de manière covalente à la surface des particules par adsorption non spécifique ou spécifique, laquelle liaison covalente aux particules est obtenue avec des groupes de surface réactifs.

16. Particules d'excipient de médicament selon la revendication 15, **caractérisées en ce que** la liaison à la particule est réalisée avec des groupes époxy ou aldéhyde ou après activation de la surface des particules avec des activateurs, en particulier carbodiimide, N-éthoxycarbonyl-2-éthoxy-1,2-dihydroquinoline, glutardialdéhyde, bromure cyanique, métaperiodate (sel Na ou sel K), chlorure de tosyle et ester d'acide chloroformique.

17. Particules d'excipient de médicament selon la revendication 16, **caractérisées en ce que** la liaison de l'apo E se fait par ses groupes amino.

18. Particules d'excipient de médicament selon les revendications 15 à 17, **caractérisées en ce que** l'apolipoprotéine E est présente en association avec une ou plusieurs autres protéines de reconnaissance, en particulier avec l'apolipoprotéine A-I et/ou A-IV.

19. Particules d'excipient de médicament selon l'une des revendications 15 à 18, **caractérisées en ce que** l'apo A-I et l'apo A-IV peuvent être utilisées seules ou en association.

20. Particules d'excipient de médicament selon l'une des revendications 15 à 19, **caractérisées en ce que** l'apo E, le cas échéant en association avec une autre protéine de reconnaissance, par rapport à l'excipient de médicament, est présente en une quantité de 0,001 à 40% en poids, en particulier de 0,01 à 30 % en poids et de préférence de 0,1 à 15 % en poids.

21. Particules d'excipient de médicament selon l'une des revendications 15 à 20, **caractérisées en ce qu'**elles sont présentes sous forme de particules solides ou de suspensions de particules, qui comprennent de préférence des nanoparticules ou des microparticules amorphes ou cristallines, ou qui sont présentes sous forme de nanosuspensions ou d'hydrosols.
